# EUROPÄISCHE PATENTSCHRIFT

(11) **EP 1 018 007 B1**
(45) Veröffentlichungstag und Bekanntmachung des Hinweises auf die Patenterteilung: **26.07.2006**
(21) Anmeldenummer: 98956830.8
(22) Anmeldetag: 21.09.1998
(51) Int. Cl.: G01N 33/50, G01N 33/53, C12Q 1/68, C07K 1/04

(54) **ADRESSIERBARES MODULARES ERKENNUNGSSYSTEM, SEINE HERSTELLUNG UND VERWENDUNG**
ADDRESSABLE MODULAR RECOGNITION SYSTEM, PRODUCTION MODE AND USE
SYSTEME DE RECONNAISSANCE MODULAIRE ADRESSABLE, SON MODE DE PRODUCTION ET SON UTILISATION

(30) Priorität: 22.09.1997 DE 19741716
(43) Veröffentlichungstag der Anmeldung: 12.07.2000
(73) Patentinhaber: Nanogen Recognomics GmbH, 65926 Frankfurt am Main (DE)
(72) Erfinder: MICULKA, Christian, D-65929 Frankfurt am Main (DE); WINDHAB, Norbert, D-65795 Hattersheim (DE); HOPPE, Hans-Ulrich, D-65929 Frankfurt am Main (DE)
(74) Vertreter: Ackermann, Joachim
(86) Internationale Anmeldenummer: PCT/EP1998/006001
(87) Internationale Veröffentlichungsnummer: WO 1999/015893

(56) Entgegenhaltungen:
- EP-A- 0 543 550
- WO-A-93/20242
- WO-A-95/04136
- WO-A-95/07289
- WO-A-96/13522
- WO-A-96/40991
- WO-A-97/27317
- WO-A-97/40385
- WO-A-97/43232
- WO-A-98/25943
- US-A- 5 188 937

## Beschreibung

Die vorliegende Erfindung betrifft ein Erkennungssystem enthaltend
(a) mindestens eine immobilisierte Bindungskomponente A mit mindestens einer Bindestelle für die Erkennungsspeziess B und
(b) mindestens eine Erkennungsspeziess B, die an die Bindungskomponente A binden kann und mindestens eine Bindestelle für ein Substrat S enthält, wobei die Bindung der Bindungskomponente A an die Erkennungsspezies B in Form eines molekularen Paarungssystems erfolgt, wobei das molekulare Paarungssystem eine Verbindung ausgewählt aus der Gruppe der Pentopyranosyl-Nucleinsäuren, Nucleinsäure mit einer oder mehreren Aminocyclohexylethansäuren-Einheiten (CNA), der peptidischen Nucleinsäuren (PNA) oder einer Nucleinsäure mit einer oder mehreren [2-Amino-4-(carboxymethyl)cyclohexyl]-Nucleobasen enthält.

Arrays sind Anordnungen von immobilisierten Erkennungsspezien, die speziell in der Analytik und Diagnostik eine wichtige Rolle bei der simultanen Bestimmung von Analyten spielen. Beispiele sind Peptide-Arrays (Fodor et al., Nature 1993, 364. 555) und Nucleinsäure-Arrays (Southern et al. Genomics 1992, 13, 1008; U.S. Patent Nr. 5.632.957).

In der experimentellen Analytik lassen Arrays durch die lokalisierte Erzeugung von Ereignissen eine besonders einfache, schnelle und reproduzierbare Datenanalyse zu. Beispiele hierfür reichen vom physikalischen Mehrkanaldetektor bis hin zu Mikrotiterplatten in der Labormedizin.

Arrays dienen auch zur Speicherung und Verarbeitung von Informationen und sind das grundlegende Konstruktionselement der Nanotechnologie.

Weitere wichtige Anwendungsbereiche sind in der Biologie. Biochemie. Medizin und Pharmakologie zu finden. So wird in EP-A1-0 461 462 ein Immunoassay beschrieben, bei dem feldartig positionierte und immobilisierte Antigene mit einem oder mehreren Antikörpern in Kontakt gebracht werden. In WO 96/01836 wird beispielsweise ein Array von DNA-Molekülen unterschiedlicher Sequenz beschrieben, der zur Detektion von Genabschnitten diente und so beispielsweise zur Diagnose pathogener Bakterien führte.

Immobilisierung durch supramolekulare Wechselwirkungen sind auch außerhalb der Array-Anwendungen bekannt. So können Träger mit Anti-Antikörpern über ein kovalent an den Träger gebundenes Antigen fixiert werden. Die Analytik von Immunoassays basiert weitgehend auf Enzym-Immunoassays (EIAs), bei denen eine enzymatisch katalysierte Reaktion die Präsenz eines Antigen-Antikörper- oder eines Antigen-Antikörper-Antiantikörper-Komplexes anzeigt. Eine der am Komplex beteiligten Einheiten ist hierbei entweder an einem Träger immobilisiert oder selbst ein Träger, z.B. in Form von Gewebsbestandteilen.

Derartige Signalverstärkungsverfahren haben jedoch insbesondere bezüglich der Verläßlichkeit der qualitativen Aussage wie auch der Quantifizierung Nachteile. Ein besonderer Nachteil von miniaturisierten Arrays sind der Aufwand und die Kosten bei der Herstellung.

Aufgabe der vorliegenden Erfindung war daher, ein Erkennungssystem zu finden, das einfach, zuverlässig, hochselektiv und zudem billig ist.

Gegenstand der vorliegenden Erfindung ist daher ein Erkennungssystem enthaltend
(a) mindestens eine immobilisierte Bindunaskomponente A mit mindestens einer Bindestelle für die Erkennungsspezies B und
(b) mindestens eine Erkennunesspezies B. die an die Bindungskomponente A binden kann und mindestens eine Bindestelle für ein Substrat S enthält, wobei die Bindung der Bindungskomponente A an die Erkennungsspezies B in Form eines molekularen Paarungssystems erfolgt.

Das molekulare Paarungssystem gemäß der vorliegenden Erfindung enthält eine Nucleinsäure und deren Analoga in Form einer Pentopyranose. Die Pentose ist ausgewählt aus einer Ribose, Arabinose, Lyxose oder Xylose. Besonders bevorzugt ist Pyranosyl-RNA (p-RNA), Nucleinsäure mit einer oder mehreren Aminocyclohexylethansäure(CNA)-Einheiten, peptidische Nucleinsäure (PNA), oder einer Nucleinsäure mit einer oder mehreren [2-Amino-4-(carboxymethyl)cyclohexyl]-Nucleobasen. Besonders bevorzugt sind Pyranosyl-Nucleinsäuren (p-NA's) und vor allem p-RNA's.

Solche Paarungssysteme sind supramolekulare Systeme nicht-kovalenter Wechselwirkung, die sich durch Selektivität. Stabilität und Reversiblität auszeichnen, und deren Eigenschaften bevorzugt thermodynamisch. d. h. durch Temperatur, pH-Wert und Konzentration beeinflußt werden. Solche Paarungssysteme können z. B. aufgrund ihrer selektiven Eigenschaften auch als "molekularer Klebstoff" für die Zusammenführung von unterschiedlichen Metallclustern zu Cluster-Verbänden mit potentiell neuen Eigenschaften verwendet werden [siehe z. B. R. L. Letsinger, et al., Nature 1996, 382, 607-9: P. G. Schultz et al.. Nature 1996. 382.609-11].

Daher ist es besonders vorteilhaft, wenn das Paarungssystem einen Komplex darstellt, der durch Assoziation der Bindungskomponente A mit der Erkennungsspezies B über nicht-kovalente Wechselwirkungen gebildet wird. Die nicht-kovalenten Wechselwirkungen sind insbesondere Wasserstoffbrücken. Salzbrücken, Stapelungen ("Stackine"), Metalligandierungen, Charge-Transfer-Komplexe und hydrophobe Wechselwirkungen.

p-NA's sind im allgemeinen zur natürlichen RNA isomere Strukturtypen, bei denen die Pentose-Einheiten in der Pyranoseform vorliegen und durch Phosphodiestergruppen zwischen den Positionen C-2' und C-4' repetifiv verknüpft sind. Unter "Nucleobase" werden dabei die kanonischen Nucleobasen A, T, U, C. G, aber auch die Paare Isoguanin/Isocytosin und 2,6-Diaminopurin/Xanthin und im Sinne der vorliegenden Erfindung auch andere Purine und Pyrimidine wie Purin. 2,6-Diaminopurin. 6-Purinthiol. Pyridin. Pyrimidin. Isoguanin, 6-Thioguanin. Xanthin. Hypoxanthin, Isocytosin. Indol. Tryptamin. N-Phthaloyltryptamin, Coffein. Theobromin. Theophyllin. Benzotriazol oder Acridin, verstanden, und vorzugsweise Ribopyranosyladenosin. Ribopyranosylguanosin. Ribopyranosylthymidin. Ribopyranosylcytosin. Ribopyranosyltryptamin oder Ribopyranosyl-N-phthalotryptamin. Ribopyranosyl-uracil oder deren [2-Amino-4-(carboxymethyl)-ribopyranosyl]-Derivate.

p-NA's. und zwar die von der Ribose abgeleitete p-RNA's, wurden zum erstenmal von Eschenmoser et al. beschrieben (siehe Pitsch. S. et al. Helv. Chim. Acta 1993. 76. 2161; Pitsch, S. et al. Helv. Chim Acta 1995, 78. 1621; Angew. Chem. 1996. 108, 1619-1623). Sie bilden ausschließlich sogenannte Watson-Crick-gepaarte. d. h. Purin-Pyrimidin- und Purin-Purin-gepaarte, antiparallele. reversibel ..schmelzende", quasi-lineare und stabile Duplices. Homochirale p-RNA-Stränge entgegengesetzten Chiralitätssinns paaren ebenfalls kontrollierbar und sind in der gebildeten Duplex streng nicht-helical. Diese für den Aufbau supramolekularer Einheiten wertvolle Spezifität hängt mit der relativ geringen Flexibilität des Ribopyranosephosphat-Rückgrats sowie mit der starken Neigung der Basenebene zur Strangachse und der hieraus folgenden Tendenz zu intercatenarer Basenstapelung im resultierenden Duplex zusammen und läßt sich letzlich auf die Teilnahme eines 2',4'-cis-disubstiturten Ribopyranoserings am Aufbau des Rückgrates zurückführen.

Diese wesentlich besseren Paarungseigenschaften machen p-NA's gegenüber DNA und RNA für die Anwendung des Aufbaus supramolekularer Einheiten zu bevorzugten Paarungssystemen. Sie bilden ein zu natürlichen Nucleinsäuren orthogonales Paarungsystem, d. h. sie paaren nicht mit in der natürlich Form vorkommenden DNA's und RNA's, was im besonderen im diagnostischen Bereich vorteilhaft ist.

p-NA's eignen sich daher besonders für die Anwendung im Bereich der Nanotechnologie, beispielsweise zur Herstellung neuer Materialien. Diagnostika und Therapeutika sowie mikroelektronischer, photonischer bzw. optoelektronischer Bauteile und für das kontrollierte Zusammenführen molekularer Species zu supramolekularen Einheiten, wie z. B. für den (kombinatorischen) Aufbau von Proteinassemblies [siehe z. B. A. Lombardi. J. W. Bryson, W. F. DeGrado, Biomoleküls (Pept. Sci.) 1997, 40. 495-504]. da p-NA's. und besonders p-RNA's Paarungssysteme bilden, die stark und thermodynamisch kontrollierbar sind. Eine weitere Anwendung ergibt sich daher gerade im diagnostischen und drug discovery-Bereich durch die Möglichkeit, funktionelle, bevorzugt biologische Einheiten wie Proteine oder DNA/RNA-Abschnitte, z. B. mit einem p-RNA-Code zu versehen, der nicht mit den natürlichen Nucleinsäuren interferiert (siehe z. B. WO93/20242).

Die Länge der Nucleinsäure und deren Analoga ist gemäß der vorliegenden Erfindung mindestens ca. 4-50, vorzugsweise mindestens ca. 4-25. insbesondere mindestens ca. 4-15. vor allem mindestens ca. 4-10 Nukleotide.

In der vorliegenden Erfindung ist die Bindungskomponente A an einem Träger immobilisiert.

Im Dokument WO9743232 ist die Immobilisierung der intermediär gebildeten kombinatorischen Aggregate an einer Oberfläche nicht beschrieben.

Unter dem Begriff "immobilisiert" versteht man im Sinne der vorliegenden Erfindung die Ausbildung einer kovalenten Bindung, quasi-kovalenten Bindung oder supramolekularen Bindung durch Assoziation von zwei oder mehreren molekularen Spezien wie linear konstituierte Moleküle, insbesondere Peptide. Peptoide. Proteine, lineare Oligo- oder Polysaccharide Nucleinsäuren und deren Analoga, oder Monomere wie Heterocyclen, insbesondere Stickstoffheterocyclen, oder nichtlinear konstituierte Moleküle wie verzweigte Oligo- oder Polysaccharide oder Antikörper und deren funktionelle Teile. Funktionelle Teile von Antikörper sind beispielsweise Fv-Fragmente (Skerra & Plückthun (1988) Science 240. 1038), einzelkettige Fv-Fragmente (scFv; Bird et al. (1988). Science 242. 423: Huston et al. (1988) Proc. Natl. Acad. Sci. U.S.A., 85. 5879) oder Fab-Fragmente (Better et al. (1988) Science 240. 1041).

Die Trägerung erfolgt somit im allgemeinen kovalent, quasi-kovalent. supramolekular oder physikalisch wie magnetisch (A. R. Shepard et al. (1997) Nucleic Acids Res.. 25. 3183-3185, Nr. 15), im elektrischen Feld oder durch einen Molekularsieb. Die Bindungskomponente A wird hierdurch entweder direkt an der Position des Trägers synthetisiert oder an bestimmte Positionen des Trägers "gelinkt". Beispiele sind Konjugations- und Trägerverfahren über Perjodatoxidation und reduktiver Aminierung der Schiffbase. N-Hydroxisuccinimidester von vorzugsweise Dicarbonsäurelinker. Ethylendaminphosphoamidatlinker. Mercapto-. Jodacetyl- oder Maleinimido-Verfahren und/oder kovalente oder nicht-kovalente Biotin-Linker-Verfahren.

Unter dem Begriff "Träger" versteht man im Sinne der vorliegenden Erfindung Material, insbesondere Chipmaterial, das in fester oder auch gelartiger Form vorliegt. Als Trägermaterialien eignen sich beispielsweise Keramik. Metall, insbesondere Edelmetall. Gläser. Kunststoffe, kristalline Materialien bzw. dünne Schichten des Trägers, insbesondere der genannten Materialien, oder (bio)molekulare Filamente wie Cellulose. Gerüstproteine.

Eine besondere Ausführungsform ist daher ein erfindungsgemäßes Erkennungssystem, bei dem die Bindungskomponente A an einen Träger über eine kovalente Bindung, quasi-kovalente Bindung oder supramolekulare Bindung durch Assoziation von zwei oder mehreren molekularen Spezien wie linearkonstituierte Moleküle, insbesondere Peptide. Peptoide. Proteine, lineare Oligo- oder Polysaccharide, Nucleinsäuren und deren Analoga, oder Monomere wie Heterocyclen, insbesondere Stickstoffheterocyclen, oder nichtlinear konstituierte Moleküle wie verzweigte Oligo- oder Polysaccharide oder Antikörper und deren funktionelle Teile wie Fv-Fragmente, einzelkettige Fv-Fragmente (scFv) oder Fab-Fragmente, immobilisiert ist.

In einer weiteren Ausführungsform ist die Bindungskomponente A an definierten Stellen des Trägers, insbesondere in Form einer Matrix, immobilisiert, wobei die definierten Stellen des Trägers vorzugsweise adressiert sind.

Gemäß dem bevorzugten Erkennungssystem wird daher ein Molekül in der mobilen (Puffer)-Phase mit der entsprechenden Komplementärsequenz nur an der Position der passenden Adresse einen supramolekularen Komplex spontan ausbilden. Sind an diese mobile Komplementäradresse durch chemische (Konjugate) oder supramolekulare Verbindungsbildung (Komplexe) weitere Einheiten mit besonderen Funktionen wie z.B. der eines Antikörpers gebunden, wird je nach verwendeten Adressenmuster auf demselben Immobilisat-Array ein unterschiedlicher Funktionsarray aufgespannt.

Die großen Vorteile eines solchen modularen Systems sind die identische einmalige Bereitstellung der Trägereinheiten für unterschiedlichste Anwendungen und die *in situ* Erzeugung nicht- haltbarer Bio-Konjugate etwa aus Proteinen. Enzymen oder lebenden Zellen und dem Paarungsrest.

Ein weiterer Vorteil ist die schrittweise Erzeugung von Substratbindungsereignis und dem meßbaren Bindungsereignis an der Trägerposition. d. h. das Substrat kann völlig ungehindert einen ersten Komplex mit der löslichen, adressierten Komponente (Erkennunssspezies B) bilden und anschließend im Raum der Trägerposition paarend an die Bindungskomponente A immobilisieren.

Es ist ferner besonders bevorzugt, wenn die Bindungskomponente A an eine Träger-Elektrode des Trägers immobilisiert ist, da beispielsweise durch eine Signalverstärkung des Impedanzverhaltens von Träger-Elektroden bei Bindungsereignissen ein elektronisch lesbares Signal erzeugt wird. Entsprechende Elektrodenprozesse sind bei R. P. Andres (1996) Science, 272. 1323-1325 und entsprechende Impedanzmessungen sind bei M. Stelzle et al. (1993) J. of Physical Chem.. 97, 2974-2981 beschrieben.

Als Erkennungsspezies B ist beispielsweise ein Biomolekül geeignet, welches z. B. ausgewählt ist aus einem Peptid. Peptoid, Protein wie Rezeptor oder funktionelle Teile davon wie die extrazelluläre Domäne eines Membran-ständigen Rezeptors. Antikörper oder funktionelle Teile davon wie Fv-Fragmente, einzelkettige Fv-Fragmente (scFv) oder Fab-Fragemente, oder Zellbestandteile wie Lipide. Glykoproteine. Filamentbestandteile, oder Viren. Virenbestandteile wie Kapside, oder Viroide, oder deren Derivate wie Acetate und deren wirksame Teile, oder Substanzbibliotheken wie Ensembles von sich strukturell unterscheidenden Verbindungen, vorzugsweise oligomere oder polymere Peptide. Peptoide. Saccharide. Nucleinsäuren.

Üblichenveise enthält das Biomolekül eine Binderegion für die Bindungskomponente A, die vorzugsweise eine der oben beschriebenen Nucleinsäuren oder deren Analoga darstellt. Im allgemeinen wird hierbei das Biomolekül an eine ausgewählte Nucleinsäure oder Analogon über einen Linker gebunden. Beispielsweise eignet sich ein Uracil-basierender Linker, bei dem vorzugsweise die 5-Position des Uracils modifiziert wurde. z. B. N-Phthaloylaminoethyluracil, aber auch ein Indol-basierender Linker, vorzugsweise Tryptaminderivate, wie z. B. N-Phthaloyltryptamin.

In einer besonderen Ausführungsform enthält die immobilisierte Bindungskomponente A verschiedene Bindestellen für verschiedene Erkennungsspezien B. wodurch verschiedene Erkennungsspezien B an der Bindungskomponente A binden können.

In einer weiteren Ausführungsform ist mindestens eine weitere Erkennungsspezies B an die Bindungskomponente A immobilisiert.

Daher ist ein weiteres erfindungsgemäßes Erkennungssystem dadurch gekennzeichnet, daß es
(a) mindestens eine immobilisierte Bindungskomponente A mit mindestens 2+n verschiedenen Bindestellen für mindestens 2+n verschiedene Erkennungsspezien B1. B2 ... Bn und eine weitere von der Erkennungsspezies B1, B2 ... Bn verschiedene Erkennungsspezies B(n+3), die an die immobilisierte Bindungskomponente A immobilisiert ist, und
(b) mindestens (n+3) verschiedene Erkennungsspezien B1. B2 ... B(n-3).
wobei n eine ganze Zahl von 0-20. vorzugsweise 0-10. insbesondere 0-5. vor allem 0 oder 1 bedeutet.

In einer weiteren Ausgestaltung stammt die Erkennungsspezies B1, B2 ... Bn aus einer Substanzbibliothek.

Zur Strukturanalyse eines Komplexes aus einer Substanzbibliothek ist es besonders vorteilhaft, wenn die Struktur der Erkennungsspezies B(n+3) bekannt ist, und/oder die verschiedenen Erkennungsspezien B dasselbe Substrat S erkennen.

Unter dem Begriff ..Substrat" versteht man im Sinne der vorliegenden Erfindung eine nichtträgergebundene Substanz, die von dem erfindungsgemäßen Erkennungssystem erkannt werden soll. Das Substrat S ist im allgemeinen ausgewählt aus Molekülen, vorzugsweise Arzneistoffe und Pflanzenschutzwirkstoffe. Metaboliten, physiologische Botenstoffe, Derivate von Leitstrukturen. Substanzen, die im menschlichen oder tierischen Körper im Fall von krankhaften Veränderungen produziert oder im erhöhten Maß produziert werden, oder Übergangszustandanaloga, oder Peptide. Peptoide. Proteine wie Rezeptoren oder funktionelle Teile davon wie die extrazelluläre Domäne eines Membran-ständigen Rezeptors. Antikörper oder funktionelle Teile davon wie Fv-Fragmente, einzelkettige Fv-Fragmente (scFv) oder Fab-Fragemente, oder Zellbestandteile wie Lipide, Glykoproteine, Filamentbestandteile, oder Viren, Virenbestandteile wie Kapside, oder Viroide, oder deren Derivate wie Acetate, oder Monomere wie Heterozyklen, insbesondere Stickstoffheterozyklen, oder nichtlinear konstituierte Moleküle wie verzweigte Oligo- oder Polysaccharide, oder Substanzbibliotheken wie Ensembles von sich strukturell unterscheidenden Verbindungen, vorzugsweise oligomere oder polymere Peptide. Peptoide. Saccharide. Nucleinsäuren. Ester. Acetale oder Monomere wie Heterocyclen. Lipide, Steroide, oder Angriffsstrukturen von Pharmaka, vorzugsweise Arzneimittelrezeptoren, spannungs-abhängige Ionenkanäle. Transporter. Enzyme oder Biosynthese-Einheiten von Mikroorganismen.

Substanzbibliotheken sind dem Fachmann aus dem Bereich der kombinatorischen Chemie bekannt. Beispiele sind die leicht zugänglichen Peptidbibliotheken, erzeugt durch Permutation der Peptidsequenz. Paaren solche Bibliotheken, entstehen völlig neue Supra-Moleküle bzw. Komplexe. Die beachtliche Anzahl möglicher Komplexe beinhaltet möglicherweise Erkennungsregionen für Substrat-Moleküle, ähnlich dem Epitop eines Antikörpers. Die Ausführungsform läßt dann ein Screening eines solchen stochastischen Bindungsereignisses zu. Ist eine der Konjugatbibliotheken an den Träger gebunden, kann durch die Codonadresse bzw. bei gleichbleibender Adresse durch seine bloße Position direkt seine Identität (z.B. die Peptidsequenz) festgelegt werden. Der Array erzeugt für einen der Paarungsstränge eine sogenannte codierte Bibliothek und vereinfacht die Komplexanalytik der supramolekularen Bibliothek.

In einer weiteren bevorzugten Ausführungsform stellt das erfindungsgemäße Erkennungssystem einen Immunoassay dar.

Ein anderer Gegenstand der vorliegenden Erfindung ist auch ein Verfahren zur Identifizierung eines Substrates S in einer Probe mit Hilfe des erfindungsgemäßen Erkennungssystems, bei dem
(a) eine Erkennungsspezies B. die das Substrat S erkennt, mit der Probe in Kontakt gebracht wird,
(b) gleichzeitig oder nacheinander mit einer immobilisierten Erkennungsspezies B in Kontakt gebracht wird, und
(c) die Bildung eines Komplexes aus immobilisierter Bindungskomponente A, Erkennungsspezies B und Substrat S nachgewiesen wird.

Insbesondere wird bei dem erfindungsgemäßen Verfahren die Bildung des Komplexes über physikalische Parameter wie Temperatur. Salze. Lösungsmittel, elektrophoretische Vorgänge gesteuert.

Im allgemeinen wird der sich gebildete Komplex über eine Markierung wie eine radioaktive oder fluoreszierende Markierung, enzymatische Markierung. Redoxmarkierung, Spinmarkierung der Erkennungsspezies B nachgewiesen, oder über den Komplex selbst, beispielsweise über Elektrodenprozesse wie über chemische Prozesse, z. B. Redoxprozesse in der Umgebung oder an der Elektrode oder über eine physikalische Meßgröße wie über Impedanzmessung oder Gleichstrommessung.

Besondere Amptifizierungs- oder Vorkonzentrierungsschritte der Substrate werden somit für viele Anwendungen nicht benötigt, was besonders vorteilhaft ist. Die chemische und physikalische Heterogenität der Positionen vor und nach den Paarungsereignissen kann zudem mit dem direktelektronischen Verfahren sehr vorteilhaft durch Parametrisierung bzw. Eichung über die Software eliminiert werden.

Das Problem, daß wichtige Substratmoleküle für solche Anwendungen Moleküle der natürlichen Paarungssysteme DNA und RNA selbst sein können und somit mit der Adressierung in störende Wechselwirkung treten würden, wird dadurch gelöst, daß besonders stabile, selektive und nicht-natürliche Paarungssysteme, wie z. B. p-NA's, verwendet werden.

Die vorliegende Erfindung bezieht sich daher auch auf ein Verfahren, mit dem Erkennungsspecies, bevorzugt natürliche DNA- oder RNA-Stränge und Proteine, dabei bevorzugt Antikörper oder funktionelle Teile von Antikörper, durch p-NA-Abschnitte, bevorzugt p-RNA-Abschnitte, eindeutig codiert werden. Diese können dann mit den zugehörigen Codons auf einem festen Träger hybridisiert werden. Damit kann auf einem festen Träger, der in Form eines Arrays mit Codons ausgestattet ist, nur durch Einstellung von Hybridisierungsbedingungen mit immer neuen Kombinationen von Erkennungsspecies an den gewünschten Positionen immer neue, diagnostisch nützliche Arrays aufgebaut werden. Wird dann der Analyt, beispielsweise eine biologische Probe wie Serum o. ä. aufgebracht, dann werden die zu detektierenden Species in einem bestimmten Muster auf dem Array gebunden, welches dann indirekt (z. B. durch Fluoreszenzmarkierung der Erkennungsspecies) oder direkt (z. B. durch Impedanzmessung am Anknüpfungspunkt der Codons) registriert wird. Dann wird die Hybridisierung durch geeignete Bedingung aufgehoben (Temperatur. Salze, Lösungsmittel, elektrophoretische Vorgänge), so daß wieder nur der Träger mit den Codons zurückbleibt. Dieser wird dann erneut mit anderen Erkennungsspecies beladen und wird z. B. für den gleichen Analyten für die Ermittlung eines anderen Musters verwendet. Die immer neue Anordnung von Erkennungsspecies im Array-Format und die Verwendung von p-NA's als Paarungssysteme ist gegenüber anderen Systemen, siehe z. B. WO 96/13522, besonders vorteilhaft.

Nach dem erfindungsgemäßen Verfahren kann in einem weiteren Schritt der Komplex aus Erkennungsspezies B und Substrat S auch isoliert werden. Hierzu wird z. B. der Komplex aus Erkennungsspezies B und Substrat S nach Einfrieren des Bindungsgleichgewichts oder kovalentes Cross-Linking von Erkennungsspezies B und Substrat S isoliert.

Das erfindungsgemäße Erkennungssystem eignet sich folglich besonders gut zum Auffinden eines Substrates S. zur Diagnose, zur Herstellung eines Katalysators und/oder zur Herstellung eines elektronischen Bauteils, insbesondere zum Auffinden, zur Optimierung und/oder zur Herstellung eines Arzneiwirkstoffes oder Pflanzenschutzwirkstoffes.

Je nach den synthetisierten Adressen können somit für unterschiedliche Fragestellungen bzw. diagnostische Probleme schnell Kits zusammengestellt werden, die auf dem existierenden Codon-Array *in situ* das Testsystem durch Paarung bildet. Bevorzugt werden Biomoleküle. z. B. ganz allgemein Zell- oder Virus-Bestandteile, ganz besonders monoklonale Antikörper oder deren funktionelle Teile.

Die folgenden Figuren sollen die Erfindung näher beschreiben, ohne sie zu beschränken.

### BESCHREIBUNG DER FIGUREN

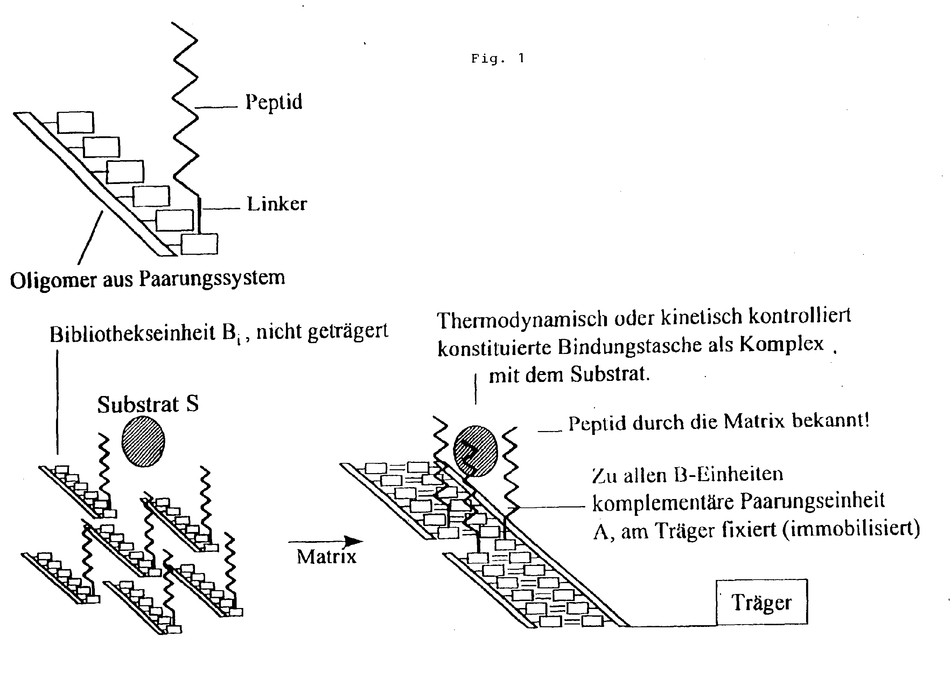

- Fig. 1: zeigt schematisch das allgemeine Prinzip einer Erkennungsspezies, die *in situ* um ein zu erkennendes Substrat erzeugt wird. Die Komplexierungseinheit (Peptid) kann durch eine Trägermatrix bekannt sein. Hierbei bildet sich eine thermodynamisch oder kinetisch kontrolliert konstituierte Bindungstasche als Komplex mit dem Substrat. Die zu allen B-Einheiten komplementäre Paarungseinheit A ist am Träger fixiert (immobilisiert).

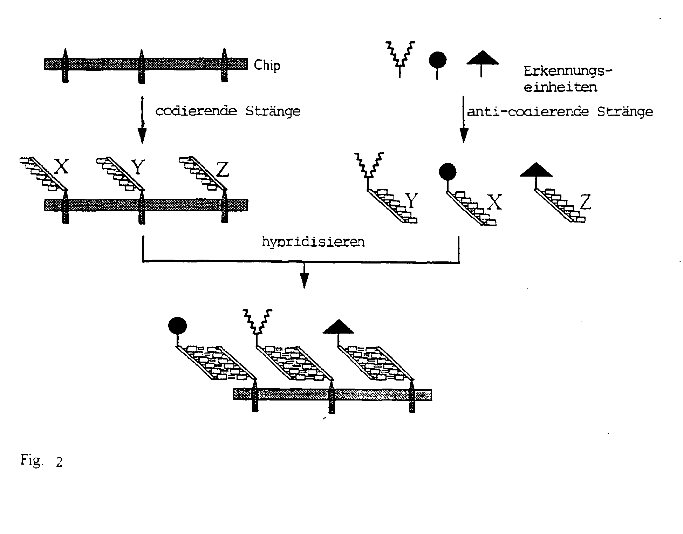

- Fig. 2: zeigt schematisch eine Anordnung von immobilisierten Erkennungsstrukturen (Arrays) auf einem festen Träger.

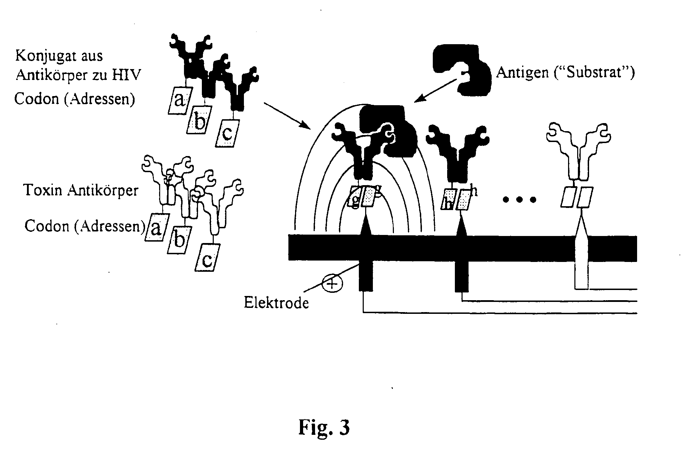

- Fig. 3: zeigt schematisch die modulare Erzeugung eines supramolekularen Arrays. Auf dem gleichen Anticodon-Träger werden durch Adressierung mit den selektiven Paarungs-regionen unterschiedliche Immunoarrays aufgebaut.

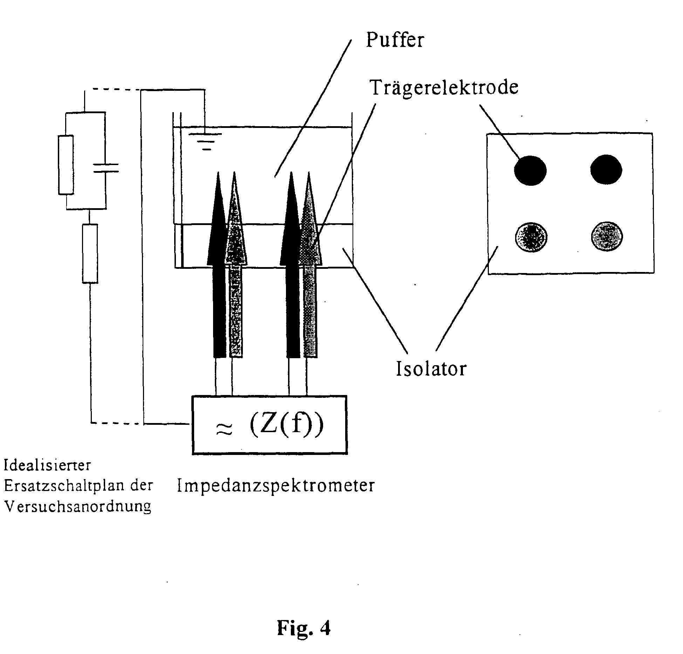

- Fig. 4: zeigt schematisch den Aufbau eines Arrays mit 4 Trägerpositionen (Elektroden) und das Meßprinzip.

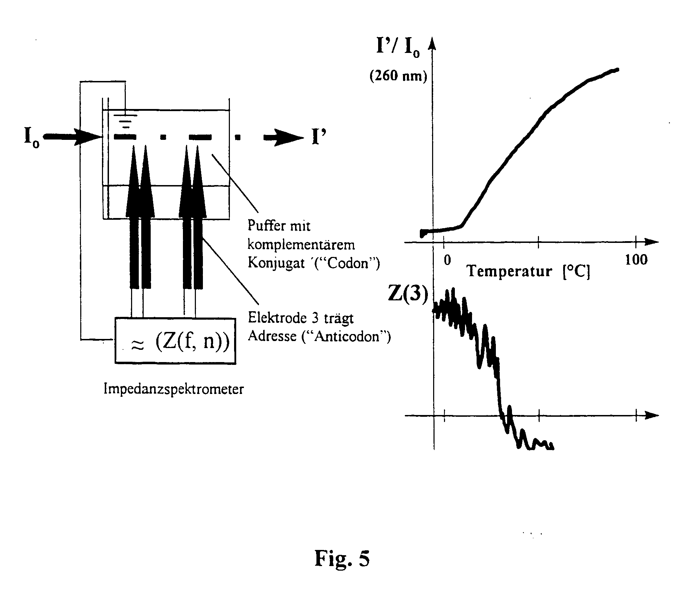

- Fig. 5: zeigt schematisch UV-spektroskopisch und Impedanz-spektroskopisch den Nachweis der Paarung der Anticodon-Codon-Moleküle. Durch Temperaturerniedrigung paaren die Stränge, der Pufferüberstand verarmt, die UV-Extinktion des Überstandes nimmt ab bzw. die Veränderung der Elektrodendoppelschicht wirkt auf die Impedanzmessung.

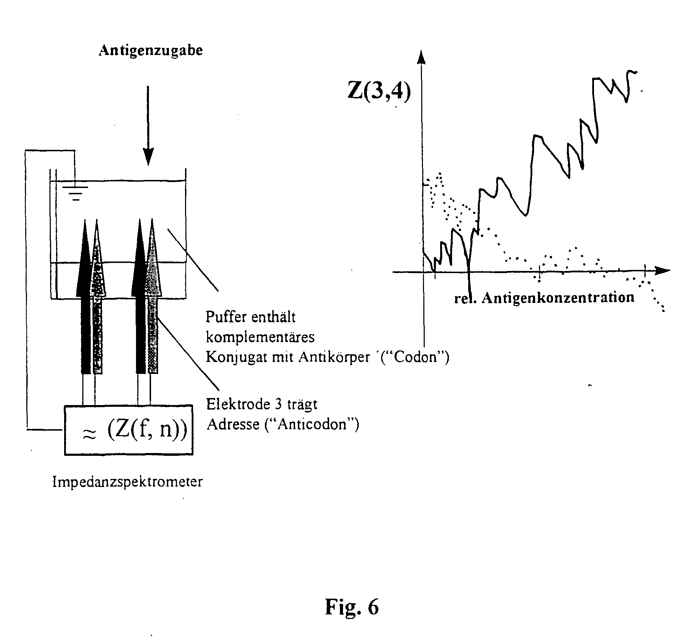

- Fig. 6: zeigt schematisch die Funktionsweise eines adressierten Immunoarrays. Lediglich Elektrode 3 trägt die passende Adresse zu einem Antikörper-Paarungsstrang-Konjugat. Wird das passende Antigen zugegeben, verändert sich die Impedanz an der Elektrode 1 anders als durch bloße Pufferveränderung an den anderen Elektroden.

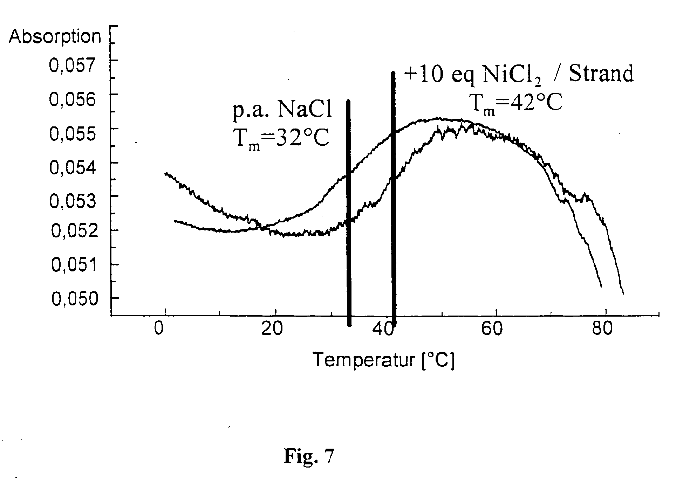

- Fig. 7: zeigt die Abkühlkurven eines temperaturinduzierten UV-Paarungsexperiments mit zwei komplementären p-RNA-Adressen, an die jeweils ein Histidin-Peptid konjugiert ist. Die Paarung erzeugt eine Erkennungsregion für Nickelionen als Substrat. Das Substrat führt zu einer deutlichen Erhöhung der Umwandlungstemperatur Tₘ, die ohne die Histidinreste nicht beobachtet wird.
- Fig. 8: zeigt schematisch eine einfache Matrix aus zwei aufgedampften Goldelektroden.
- Fig. 9: zeigt den direktelektronischen Nachweis eines Antigen-Antikörperkomplexes auf einer Elektrodenposition des Arrays durch Impedanzspektroskopie.
- Fig. 10: zeigt einen zusätzlichen Nachweis des Antigen-Antikörperkomplexes auf der adessierten Elektrode mittels Fluoreszens.

### Beispiele

### Beispiel 1

Synthese eines einen Linker enthaltenden p-RNA-Oligonucleotids mit Linker der Formel 4' AGGCAIndT2':

### 1.1 Festphasensynthese des Oligonucleotids

A, G. C. T steht für die Nucleobasen Adenin. Guanin, Cyosin und Thymin und Ind bedeutet Aminoethylindol (Indol CH₂-CH₂-NH₂) als Linker in Form einer Nucleobase.

Die vollautomatische Festphasensynthese wurde mit jeweils 15 µmol durchgeführt. Ein Synthesezyklus besteht aus den folgenden Schritten:
(a) Detritvlierung: 5 Minuten mit 6% DCA (Dichloressigsäure) in CH₂Cl₂ (79 ml).
(b) Waschen mit CH₂Cl₂ (20 ml), Acetonitril (20 ml) und danach Spülen mit Argon:
(c) Kupplung: Waschen des Harzes mit dem Aktivator (0,5 M Pyridin.HCl in CH₂Cl₂ (0,2 ml) und anschließend 30minütiges Behandeln mit Aktivator (0.76 ml) und Phosphoramidit der entsprechenden Nucleobase (0, 76 ml : 8 eq; 0.1 M in Acetonitril) im Verhältnis 1/1;
(d) Capping: 2-minütiges Behandeln mit 50% Cap A (10.5 ml) und 50% Cap B (10.5 ml) von PerSeptive Biosystems. Inc.. Texas, USA (Cap A: THF. Lutidine. Acetanhydrid: Cap B: 1-Methylimidazol. THF, Pyridin);
(e) Oxidation: 1-minütiges Behandeln mit 120 ml Iodlösung (400 mg Jod in 100 ml Acetonitril. 46 ml H₂O und 9,2 ml sym-Collidine); und
(f) Waschen mit Acetonitril (22 ml).

Zur Erleichterung der nachfolgenden HPLC Reinigung der Oligonucleotide wurde die letzte DMT(Dimethoxytrityl)-Gruppe nicht abgespalten. Zum Nachweis der letzten Kupplung mit den modifizierten Phorphoramiditen wurde nach der Synthese mit 1% des Harzes eine Tritylkationabsorption in UV (503 nm) durchgeführt.

### 1.2 Aufarbeitung des Oligonucleotids:

Die Abspaltung der Allyletherschutzgruppen erfolgte mit einer Lösung von Tetrakis(triphenylphosphin)palladium (272mg), Triphenylphosphin (272 mg) und Diethylammoniumhydrogencarbonat in CH₂Cl₂ (15ml) nach 5 Stunden bei RT. Die Glasträger wurden danach mit CH₂Cl₂ (30ml), Aceton (30ml) und Wasser (30ml) gewaschen. Um Palladiumkomplexreste zu entfernen, wurde das Harz mit einer wäßrigen 0,1 M Natriumdiethyldithiocarbamathydratlösung gespült. Die obenerwähnte Waschoperation wurde in einer umgekehrten Reihe noch einmal durchgeführt. Anschließend wurde das Harz am Hochvakuum 10 Minuten getrocknet. Der Abspaltungsschritt vom Glasträger bei gleichzeitiger Debenzovlierung wurde in 24% Hydrazinhydratlösung (6ml) bei 4°C durchgeführt. Nach HPLC-Kontrolle an RP 18 (18-25 Stunden) wurde das Oligonucleotid .,Trityl ON" mittels einer aktivierten (Acetonitril, 20 ml) Waters Sep-Pak Cartridge vom Hydrazin befreit. Das Hydrazin wurde mit TEAB, 0,1 M (30ml) gewaschen. Das Oligonucleotid wurde dann mit Acetonitril/TEAB. 0.1 M (10ml) eluiert. Anschließend wurde mittels HPLC zur Abtrennung von Abbruchsequenzen gereinigt und die DMT-Entschützung (30 ml 80%ig wäßrige Ameisensäure) durchgeführt. Abschlie-βende Entsalzung (über Sep-Pak Kartouche, mit TEAB Puffer 0,1 M/Acetonitril: 1'1) lieferte das reine Oligonucleotid.

### Beispiel 2

### Jodacetylierung von p-RNA mit N-(Jodacetyloxy)-succinimid

*p*-RNA-Sequenz : 4' AGGCAIndT 2' M_{w} = 2266.56 g/mol, hergestellt gemäß Beispiel 1.

1 eq. der *p*-RNA wurde in einer 0.1 molaren Natriumhydrogencarbonatlösung (pH 8.4) gelöst (1 ml pro 350 nmol) und mit einer Lösung von N-(Jodacetyloxy)-succinimid in DMSO versetzt (40 ul pro mg). Man dunkelt den Ansatz mit Aluminiumfolie ab und ließ ihn bei Raumtemperatur für 30-90 Minuten stehen.

Der Fortgang der Reaktion wurde mittels analytischer HPLC verfolgt. Die Standardbedingungen waren:
Puffer A : 0.1 molarer Triethylammoniumacetat-Puffer in Wasser
Puffer B : 0.1 molarer Triethylammoniumacetat-Puffer in Wasser:Acetonitril 1:4
Gradient : von 10% B startend auf 50% B in 40 Minuten
Säulenmaterial : 10 µM LiChrosphere® 100 RP-18 von Merck Darmstadt GmbH; 250 x 4 mm
Retentionszeit der Edukte: 18,4 Minuten
Retentionszeit der Produkte in diesem Falle : 23,1 Minuten

Nach beendeter Reaktion wurde der Ansatz mit Wasser auf das vierfache Volumen verdünnt. Man aktivierte eine Waters Sep-Pak-Kartusche RP-18 (ab 15 oD 2 g Füllung) mit 2 x 10 ml Acetonitril und 2 x 10 ml Wasser, trug das Oligonucleotid auf, ließ einsinken, wusch das Reaktionsgetaß mit 2 x 10 ml Wasser, wusch mit 3 x 10 ml Wasser nach, um Salz und Reagenz zu entfernen, und eluierte zuerst mit 5 x 1 ml 50:1 Wasser : Acetonitril und anschließend mit 1:1. Das Produkt eluierte in den 1:1-Fraktionen in sehr guter Reinheit. Die Fraktionen wurden in der Kälte und im Dunkeln eingeengt, vereinigt, und wieder eingeengt.

Die Ausbeuten wurden mittels UV-Absorptionspektrometrie bei 260 nm bestimmt.

### Massenspektrometrie :

| | | |
|---|---|---|
| Sequenz : | 4' AGGCAInd(CH₂CH₂NHCOCH₂-I)T2' | |
| | berechnete Masse : | 2434.50 g/mol |
| | gefundene Masse MH₂²⁺: | 1217.9 g/mol = 2433 |

### Beispiel 3

Konjugation von *p*-RNA an ein Peptid der Sequenz (His)₆:

Die jodacetylierte *p*-RNA (M_{w} = 2434.50 g/mol) wurde in einem Puffersystem gelöst (1000µl pro 114 nmol) und dann mit einer Lösung des Peptides in Puffer versetzt (2 moleq. (His)₆₋Peptid).
Puffersystem : Borax/HCl-Puffer der Firma Riedel-de Haën. pH 8,0. wurde im Verhältnis 1:1 mit einer 10 millimolaren Lösung von EDTA-Dinatriumsalz in Wasser gemischt und auf pH 6,3 mit HCl eingestellt. Man erhielt dadurch eine Lösung, die 5 mM Na₂EDTA enthält.

Man beließ den Ansatz bis zum vollständigen Umsatz bei Raumtemperatur im Dunkeln. Die Reaktion wurde mittels HPLC-Analytik verfolgt. Nach beendeter Reaktion wurde der Ansatz direkt mittels RP-HPLC gereinigt. Die Fraktionen wurden in der Kälte und im Dunkeln eingeengt, vereinigt, und wieder eingeengt. Man nahm in Wasser auf und entsalzte. Man aktivierte eine Waters Sep-Pak-Kartusche RP-18 (ab 15 oD 2 g Füllung) mit 2 x 10 ml Acetonitril und 2 x 10 ml Wasser, trug das Oligonucleotid auf, ließ einsinken, wusch das Reaktionsgefäß mit 2 x 10 ml Wasser, wusch mit 3 x 10 ml Wasser nach, um das Salz zu entfernen, und eluierte mit Wasser : Acetonitril 1:1. Die Produkt-Fraktionen wurden eingeengt, vereinigt, und wieder eingeengt.
Die Ausbeuten wurden mittels UV-Absorptionspektrometrie bei 260 nm bestimmt. Sie erreichten 70-95% der Theorie.

HPLC-Analytik:
Puffer A : 0,1 molarer Triethylammoniumacetat-Puffer in Wasser
Puffer B : 0,1 molarer Triethylammoniumacetat-Puffer in Wasser:Acetonitril 1:4
Gradient: von 10% B startend auf 50% B in 40 Minuten
Säulenmaterial : 10 µM LiChrosphere ® 100 RP-18 von Merck Darmstadt GmbH: 250 x 4
Retentionszeit des Produktes : 16.9 Minuten

### Massenspektrometrie :

| | | |
|---|---|---|
| Sequenz : | 4' AGGCAInd(CH₂CH₂NHCOCH₂-(His)₆T 2' | |
| | berechnete Masse : MH₂²⁺ : | 1626,9 g/mol |
| | gefundene Masse MH₂²⁺ : | 1626,0 g/mol |

Analog wurde die komplementäre Sequenz 4' Ind(CH₂CH₂NHCOCH₂-(His)₆TGCCT2 hergestellt:

| | |
|---|---|
| berechnete Masse MH₂^{2*}: | 1436,2 g/mol |
| gefundene Masse MH₂^{2*}: | 1436,4 g/mol |

Analog wurden auch Peptidbibliotheken zur Bildung von Erkennungsregionen an die p-RNA konjugiert.

Im UV-Lösungsexperiment konnte nachgewiesen werden, daß die Wechselwirkung der Histidinuntereinheiten mit einem Substrat (Nickel-Ionen), die Paarungseigenschaften selbst beeinflussen. Eine je 5µM p-RNA-Konjugatlösung in 10mM Tris HCl 150mM ultrarein NaCl zeigte im UV-Paarunesexperiment ein Tₘ von 32°C. der sich nach Zugabe von 10 Äquivalenten Nickel-Ionen pro Strang um 10° C auf 42° erhöhte. Somit geht der Nachweis. d. h. die Erkennung eines Substrates hier sehr vorteilhaft mit der Adressierung selbst einher: das entspricht auf der Trägermatrix dem Immobilisierungsvorgang.

### Beispiel 4

### Direktelektronische Detektion einer Antikörper/Antigen-Erkennung auf dem adressierbaren Erkennungssystem

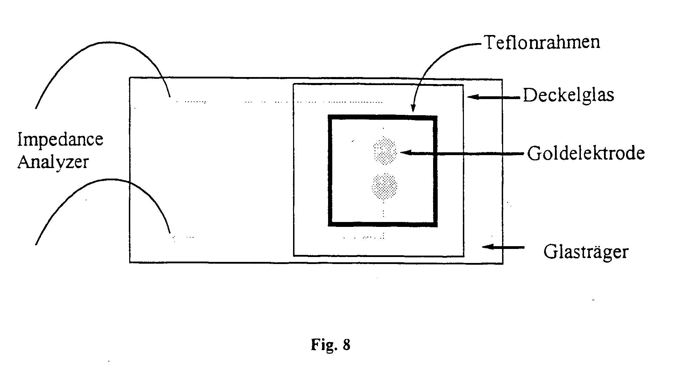

Eine einfache Matrix aus zwei aufgedampften Goldelektroden wurde als Beispiel eines adressierbaren Erkennungssystems verwendet (siehe Fig. 8).

An eine jodacetylierte p-RNA Sequenz wurde eine im Handel erhältliche Thiol-reduzierte Antikörpereinheit (Rockland Immunochemicals. Pennsylvania, USA) wie oben beschrieben ankonj ugiert.

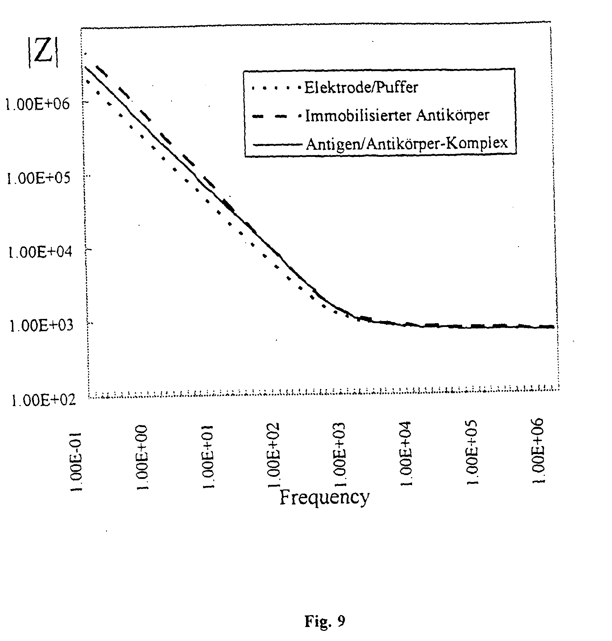

Die komplementäre p-RNA-Einheit 4' Ind--TAGGCAA T 2' wurde mittels 100 equivalenten Traut's Reagenz in 1mM wässriger EDTA und Borax Puffer pH 9.5 am Aminoiinker Thiolaktiviert, nach 6 Stunden reversed-phase-HPL-chomatographisch gereinigt, und über Nacht an einer der beiden frisch mittels UV-licht gereinigten Gold-Elektroden gebunden. Nur diese Elektrode bindet das Antikörper-p-RNA-Konjugat durch Paarung (siehe Fig. 9).

Die Figur zeigt das Impedanz-Signal (ohne weitere Beschaltung; Spektrometer Solarton Instruments 1260 interface; Solarton Instruments SI 1287) des thio-reduzierten Antikörpers, der direkt über Nacht auf eine frisch gereinigte Elektrode der beschriebenen Art gebunden wurde vor und nach einer Antikörper-Antigen-Komplexierung des immobilisierten Antikörpers unter den Pufferbedingungen 1/15 mol/l Na₂HPO₄, KH₂PO₄, pH 7.4 und Raumtemperatur.

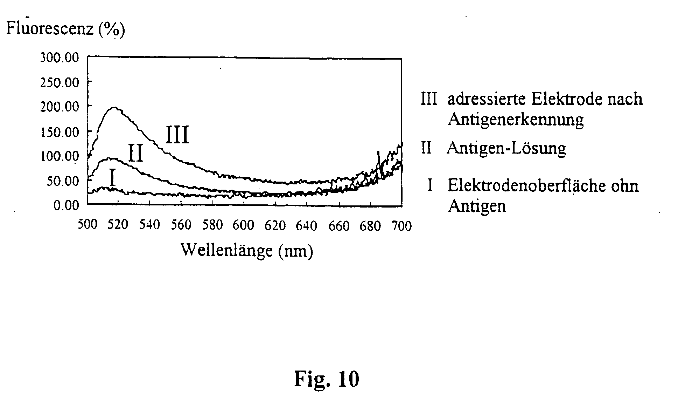

Das Erkennungs-Ergebnis konnte im gewählten Fall mittels Fluoreszenzmarker geprüft werden, da das im Handel erhältliche Antigen (eine Human-IgG-F(ab')2-Fraktion Rockland Immunochemicals) Fluorescein-markiert ist (siehe Fig. 10).

## Patentansprüche

1. Erkennungssystem enthaltend
(a) mindestens eine immobilisierte Bindungskomponente A mit mindestens einer Bindestelle für die Erkennungsspezies B und
(b) mindestens eine Erkennungsspezies B, die an die Bindungskomponente A binden kann und mindestens eine Bindestelle für ein Substrat S enthält, **dadurch gekennzeichnet, daß** die Bindung der Bindungskomponente A an die Erkennungsspezies B in Form eines molekularen Paarungssystems erfolgt, wobei das molekulare Paarungssystem eine Verbindung ausgewählt aus der Gruppe der Pentopyranosyl-Nucleinsäuren, Nucleinsäure mit einer oder mehreren Aminocyclohexylethansäuren-Einheiten (CNA), der peptidischen Nucleinsäuren (PNA) oder einer Nucleinsäure mit einer oder mehreren [2-Amino-4-(carboxymethyl)cyclohexyl]-Nucleobasen enthält.

2. Erkennungssystem nach Anspruch 1, **dadurch gekennzeichnet, daß** die Pentopyranosyl-Einheit der Pentopyranosyl-Nucleinsäure eine Ribose, Arabinose, Lyxose oder Xylose ist.

3. Erkennungssystem nach Anspruch 2, **dadurch gekennzeichnet, daß** die Pentopyranosyl-Nucleinsäure eine Pyranosyl-RNA (p-RNA) ist.

4. Erkennungssystem nach einem der Ansprüche 1-3, **dadurch gekennzeichnet, daß** die Nukleobase-Einheit des molekularen Paarungssystemsausgewählt sind aus Purin, 2.6-Diaminopurin, 6-Purinthiol, Pyridin, Pyrimidin, Adenin, Guanin, Isoguanin, 6-Thioguanin, Xanthin, Hypoxanthin, Thymidin, Cytosin, Isocytosin, Indol, Tryptamin, N-Phthaloyltryptamin, Uracil, Coffein, Theobromin, Theophyllin. Benzotriazol oder Acridin.

5. Erkennungssystem nach einem der Ansprüche 1-4, **dadurch gekennzeichnet, daß** die Pyranosyl-Nucleotide ausgewählt sind aus Ribopyranosyladenosin, Ribopyranosylguanosin, Ribopyranosylthymidin, Ribopyranosylcytosin, Ribopyranosyltryptamin oder Ribopyranosyl-N-phthalotryptamin, Ribopyranosyl-uracil oder deren [2 Amino-4-(carboxymethyl)ribopyranosyl]-Derivate.

6. Erkennungssystem nach einem der Ansprüche 1-5, **dadurch gekennzeichnet, daß** die Länge der Pentopyranosyl-Nucleinsäure 4-50 Nukleotide ist.

7. Erkennungssystem nach einem der Ansprüche 1-6, **dadurch gekennzeichnet, daß** die Bindungskomponente A an einem Träger immobilisiert ist.

8. Erkennungssystem nach Anspruch 7, **dadurch gekennzeichnet, daß** der Träger ausgewählt ist aus der Gruppe der Keramiken, Metalle, Gläser, Kunststoffe, kristallinen Materialien oder dünnen Schichten des Trägers der genannten Materialien, oder der (bio)molekularen Filamente, Cellulose, Gerüstproteine.

9. Erkennungssystem nach Anspruch 7 oder 8, **dadurch gekennzeichnet, daß** die Bindungskomponente A an einen Träger über eine kovalente Bindung, quasi-kovalente Bindung oder supramolekulare Bindung durch Assoziation von zwei oder mehreren molekularen Spezien wie linearkonstituierte Moleküleausgewählt aus der Gruppe der Peptide, Peptoide, Proteine, lineare Oligo- oder Polysaccharide, Nucleinsäuren und deren Analoga, oder heterocyclische Monomere, oder nichtlinear konstituierte Moleküle ausgewählt aus der Gruppe der verzweigten Oligo- oder Polysacharide oder Antikörper und deren funktionelle Teile ausgewählt aus der Gruppe der Fv-Fragmente, einzelkettigen Fv-Fragmente (scFv) oder Fab-Fragmente, immobilisiert ist.

10. Erkennungssystem nach einem der Ansprüche 7-9, **dadurch gekennzeichnet, daß** die Bindungskomponente A an definierten Stellen des Trägers, vorzugsweise in Form einer Matrix, immobilisiert ist.

11. Erkennungssystem nach Anspruch **10, dadurch gekennzeichnet, daß** die definierten Stellen des Trägers adressiert sind.

12. Erkennungssystem nach einem der Ansprüche 7-11, **dadurch gekennzeichnet, daß** die Bindungskomponente A an einer Träger-Elektrode des Trägers immobilisiert ist.

13. Erkennungssystem nach einem der Ansprüche 1-112 **dadurch gekennzeichnet, daß** die Erkennungsspezies B ein Biomolekül ist.

14. Erkennungssystem nach Anspruch 13, **dadurch gekennzeichnet, daß** das Biomolekül ausgewählt ist aus der Gruppe der Peptide, Peptoide, Proteine oder funktionellen Teilen davon, Antikörpern oder funktionellen Teilen davon, Fv-Fragmente, einzelkettigen Fv-Fragmente (scFv) oder der Fab-Fragemente, oder Zellbestandteile, Lipide, Glykoproteine, Filamentbestandteile oder Viren, Virenbestandteile, Kapside, oder Viroide, oder deren Derivate, und deren wirksame Teile, oder Substanzbibliotheken aus Ensembles von sich strukturell unterscheidenden Verbindungenaus der Gruppe der oligomeren oder polymeren Peptide, Peptoide, Saccharideund Nucleinsäuren.

15. Erkennungssystem nach einem der Ansprüche 1-14, **dadurch gekennzeichnet, daß** die immobilisierte Bindungskomponente A verschiedene Bindestellen für verschiedene Erkennungsspezien B enthält, wodurch verschiedene Erkennungsspezien B an der Bindungskomponente A binden können.

16. Erkennungssystem nach einem der Ansprüche 1-15, **dadurch gekennzeichnet, daß** mindestens eine weitere Erkennungsspezies B an die Bindungskomponente A immobilisiert ist.

17. Erkennungssystem nach Anspruch 15 oder 16, **dadurch gekennzeichnet, daß** es
(a) mindestens eine immobilisierte Bindungskomponente A mit mindestens 2+n verschiedenen Bindestellen für mindestens 2+n verschiedene Erkennumgsspezien B1, B2 ... Bn und eine weitere von der Erkennungsspezies B1, B2 ... Bn verschiedene Erkennungsspezies B(n+3), die an die immobilisierte Bindungskomponente A immobilisiert ist, und
(b) mindestens (n+3) verschiedene Erkennungsspezien B1, B2 ... B(n+3),
wobei n eine ganze Zahl von 0-20, vorzugsweise 0-10, insbesondere 0-5, vor allem 0 oder 1 bedeutet.

18. Erkennungssystem nach Anspruch 17, **dadurch gekennzeichnet, daß** die Erkennungsspezies B1, B2 ... Bn aus einer Substanzbibliothek stammt.

19. Erkennungssystem nach Anspruch 17 oder 18, **dadurch gekennzeichnet, daß** die Struktur der Erkennungsspezies B(n+3) bekannt ist.

20. Erkennungssystem nach einem der Ansprüche 15-19, **dadurch gekennzeichnet, daß** die verschiedenen Erkennungsspezien B dasselbe Substrat S erkennen.

21. Erkennungssystem nach einem der Ansprüche 1-20, **dadurch gekennzeichnet, daß** das Substrat S ausgewählt ist aus der Gruppe derArzneistoffe und Pflanzenschutzwirkstoffe, der Metaboliten, der physiologischen Botenstoffe, Derivate von Leitstrukturen, Substanzen, die im menschlichen oder tierischen Körper im Fall von krankhaften Veränderungen produziert oder im erhöhten Maß produziert werden, oder Übergangszustandanaloga, oder Peptide, Peptoide, Proteine wie Rezeptoren oder funktionelle Teile davon, Antikörper oder funktionelle Teile davon, Fv-Fragmente, einzelkettige Fv-Fragmente (scFv) oder Fab-Fragemente, oder Zellbestandteile, Lipide, Glykoproteine, Filamentbestandteile, oder Viren, Virenbestandteile, Kapside, oder Viroide, oder deren Derivate, oder heterocyclische Monomere, oder nichtlinear konstituierte Moleküle aus der Gruppe der verzweigten Oligo- oder Polysaccharide, oder Substanzbibliotheken aus Ensembles von sich strukturell unterscheidenden Verbindungen, oligomere oder polymere Peptide, Peptoide, Saccharide, Nucleinsäuren, Ester, Acetale oder heterocyclische Monomere, Lipide, Steroide, oder Angriffsstrukturen von Pharmaka, Arzneimittelrezeptoren, spannungs-abhängige Ionenkanäle, Transporter, Enzyme oder Biosynthese-Einheiten von Mikroorganismen.

22. Erkennungssystem nach einem der Ansprüche 1-21, **dadurch gekennzeichnet, daß** es einen Immunoassay darstellt.

23. Verfahren zur Identifizierung eines Substrates S in einer Probe mit Hilfe des Erkennungssystems gemäß einem der Ansprüche 1-22, **dadurch gekennzeichnet, dass**
(a) eine Erkennungsspezies B, die das Substrat S erkennt, mit der Probe in Kontakt gebracht wird,
(b) gleichzeitig oder nacheinander mit einer immobilisierten Erkennungsspezies B in Kontakt gebracht wird, und
(c) die Bildung eines Komplexes aus immobilisierter Bindungskomponente A, Erkennungsspezies B und Substrat S nachgewiesen wird.

24. Verfahren nach Anspruch 23, **dadurch gekennzeichnet, daß** die Bildung des Komplexes über physikalische Parameter wie Temperatur, Salze, Lösungsmittel, elektrophoretische Vorgänge gesteuert wird.

25. Verfahren nach Anspruch 23 oder 24, **dadurch gekennzeichnet, daß** der Komplex über eine eine radioaktive oder fluoreszierende Markierung, enzymatische Markierung, Redoxmarkierung, Spinnmarkierung der Erkennungsspezies B nachgewie-gen wird, oder über den Komplex selbst,über Elektrodenprozesse, chemische Prozesse, Redoxprozesse in der Umgebung oder an der Elektrode oder über eine physikalische Meßgröße, Impedanzmessung oder Gleichstrommessung nachgewiesen wird.

26. Verfahren nach einem der Ansprüche 23-25, **dadurch gekennzeichnet, daß** in einem weiteren Schritt der Komplex aus Erkennungsspezies B und Substrat S isoliert wird.

27. Verfahren nach Anspruch 26, **dadurch gekennzeichnet, daß** der Komplex aus Erkennungsspezies B und Substrat S nach Einfrieren des Bindungsgleichgewichts oder kovalentes Cross-Linking von Erkennungsspezies B und Substrat S isoliert wird.

28. Verwendung des Erkennungssystems gemäß einem der Ansprüche 1-22 zum Auffinden eines Substrates S, zur Diagnose, zur Herstellung eines Katalysators und/oder zur Herstellung eines elektronischen Bauteils, zum Auffinden oder zur Optimierung und/oder zur Herstellung eines Arzneiwirkstoffes oder Pflanzenschutzwirkstoffes.

## Claims

1. Recognition system comprising
(a) at least one immobilized binding component A having at least one binding site for the recognition species B and
(b) at least one recognition species B which can bind to the binding component A and contains at least one binding site for a substrate S, **characterized in that** the binding of the binding component A to the recognition species B takes place in the form of a molecular pairing system, wherein the molecular pairing system comprise a compound selected from pyranosyl- nucleic acids, nucleic acid having one or more amino-cyclohexylethanoic acid (CNA) units, peptide nucleic acid (PNA), or a nucleic acid having one or more [2-amino-4-(carboxymethyl)cyclohexyl]-nucleobases.

2. Recognition system according to Claim 1, **characterized in that** the pentopyranosyl moiety of the pentopyranosyl- nucleic acid is a ribose, arabinose, lyxose or xylose.

3. Recognition system according to Claims 2, **characterized in that** the pentopyranosyl- nucleic acid is a pyranosyl-RNA (p-RNA).

4. Recognition system according to one of Claims 1-3, **characterized in that** the pentopyranosyl nucleotides are selected from purine, 2,6-diaminopurine, 6-purinethiol, pyridine, pyrimidine, adenine, guanine, isoguanine, 6-thioguanine, xanthine, hypoxanthine, thymidine, cytosine, isocytosine, indole, tryptamine, N-phthaloyltryptamine, uracil, caffeine, theobromine, theophylline, benzotriazole or acridine.

5. Recognition system according to one of Claims 1-4, **characterized in that** the pentopyrtanosyl- nucleic acid is selected from ribopyranosyladenosine, ribopyranosylguanosine, ribopyranosylthymidine, ribopyranosylcytosine, ribopyranosyltryptamine or ribopyranosyl-N-phthalotryptamine, ribopyranosyl-uracil or their 2-amino-4-(carboxymethyl)ribopyranosyl] derivatives.

6. Recognition system according to one of Claims 1-5, **characterized in that** the length of the pentopyranosyl- nucleic acid and its analogues is 4-50 nucleotides.

7. Recognition system according to one of Claims 1-6, **characterized in that** the binding component A is immobilized on a carrier.

8. Recognition system according to Claim 7, **characterized in that** the carrier is selected from the group of ceramics, metals, in particular noble metals, glasses, plastics, crystalline materials or thin layers of the carrier of the materials mentioned, or (bio)molecular filaments, cellulose, structural proteins.

9. Recognition system according to Claim 7 or 8, **characterized in that** the binding component A is immobilized on a carrier by means of a covalent bond, quasi-covalent bond or supramolecular bond by association of two or more molecular species such as molecules of linear constitution selected from the group of peptides, peptoids, proteins, linear oligo- or polysaccharides, nucleic acids and their analogues, or heterocyclic monomers, or molecules of non-linear constitution selected from the group of branched oligo- or polysaccharides or antibodies and their functional moieties selected from the group of Fv fragments, single-chain Fv fragments (scFv) or Fab fragments.

10. Recognition system according to one of Claims 7-9, **characterized in that** the binding component A is immobilized at defined sites of the carrier, preferably in the form of a matrix.

11. Recognition system according to Claim 10, **characterized in that** the defined sites of the carrier are addressed.

12. Recognition system according to one of Claims 7-11, **characterized in that** the binding component A is immobilized on a carrier electrode of the carrier.

13. Recognition system according to one of Claims 1-12, **characterized in that** the recognition species B is a biomolecule.

14. Recognition system according to Claim 17, **characterized in that** the biomolecule is selected from the group of peptides, peptoids, proteins or functional moieties thereof, antibodies or functional moieties thereof, Fv fragments, single-chain Fv fragments (scFv) or Fab fragments, or cell constituents such as lipids, glycoproteins, filament constituents, or viruses, viral constituents such as capsids, or viroids, or their derivatives and their active moieties, or substance libraries from ensembles of structurally differing compounds selected from the group of oligomeric or polymeric peptides, peptoids, saccharides and nucleic acids.

15. Recognition system according to one of Claims 1-14, **characterized in that** the immobilized binding component A contains various binding sites for various recognition species B, by means of which various recognition species B can bind to the binding component A.

16. Recognition system according to one of Claims 1-15, **characterized in that** at least one further recognition species B is immobilized on the binding component A.

17. Recognition system according to Claim 15 or 16, **characterized in that** it comprises
(a) at least one immobilized binding component A having at least 2+n different binding sites for at least 2+n different recognition species B1, B2 ... Bn and a further recognition species B(n+3) different from the recognition species B1, B2 ... Bn, which is immobilized on the immobilized binding component A, and
(b) at least (n+3) different recognition species B1, B2 ... B(n+3),
where n is an integer from 0-20, preferably 0-10, in particular 0-5, especially 0 or 1.

18. Recognition system according to Claim 17, **characterized in that** the recognition species B1, B2 ... Bn originates from a substance library.

19. Recognition system according to Claim 17 or 18, **characterized in that** the structure of the recognition species B(n+3) is known.

20. Recognition system according to one of Claims 15-19, **characterized in that** the different recognition species B recognize the same substrate S.

21. Recognition system according to one of Claims 1-20, **characterized in that** the substrate S is selected from the group of pharmaceuticals and plant protection active compounds, metabolites, physiological messenger substances, derivatives of lead structures, substances which are produced or produced to an increased extent in the human or animal body in the case of pathological changes, or transition state analogues, or peptides, peptoids, proteins such as receptors or functional moieties thereof, antibodies or functional moieties thereof, Fv fragments, single-chain Fv fragments (scFv) or Fab fragments, or cell constituents, lipids, glycoproteins, filament constituents, or viruses, viral constituents, capsids, or viroids, or their derivatives such as acetates, or heterocyclic monomers, or molecules of non-linear constitution selected from the group of branched oligo- or polysaccharides, or substance libraries of ensembles of structurally differing compounds, oligomeric or polymeric peptides, peptoids, saccharides, nucleic acids, esters, acetals or heterocyclic monomers, lipids, steroids, or target structures for pharmaceuticals, pharmaceutical receptors, voltage-dependent ion channels, transporters, enzymes or biosynthesis units of microorganisms.

22. Recognition system according to one of Claims 1-21, **characterized in that** it is an immunoassay.

23. Process for the identification of a substrate S in a sample with the aid of the recognition system according to one of Claims 1-22, **characterized in that**
(a) a recognition species B which recognizes the substrate S is brought into contact with the sample,
(b) is simultaneously or successively brought into contact with an immobilized recognition species B, and
(c) the formation of a complex of immobilized binding component A, recognition species B and substrate S is detected.

24. Process according to Claim 23, **characterized in that** the formation of the complex is controlled by means of physical parameters such as temperature, salts, solvents, electrophoretic processes.

25. Process according to Claim 23 or 24, **characterized in that** the complex is detected by radioactive or fluorescent labelling, enzymatic labelling, redox labelling, spin labelling of the recognition species B, or by means of the complex itself, by electrode processes, by means of chemical processes, by redox processes in the environment or on the electrode or by means of a physical parameter such as by means of impedance measurement or direct current measurement.

26. Process according to one of Claims 23-25, **characterized in that** the complex of recognition species B and substrate S is isolated in a further step.

27. Process according to Claim 26, **characterized in that** the complex of recognition species B and substrate S is isolated after freezing the binding equilibrium or covalent cross-linking of recognition species B and substrate S.

28. Use of the recognition system according to one of Claims 1-22 for finding a substrate S for diagnosis, for the preparation of a catalyst and/or for the preparation of an electronic component, for the finding or for the optimization and/or for the preparation of a pharmaceutical active compound or plant protection active compound.

## Revendications

1. Système de reconnaissance contenant :
(a) au moins un composant de liaison A immobilisé avec au moins une position de liaison pour l'espèce de reconnaissance B et
(b) au moins une espèce de reconnaissance B, qui peut se lier au composant de liaison A et contient au moins une position de liaison pour un substrat S, **caractérisé en ce que** la liaison du composant de liaison A à l'espèce de reconnaissance B s'effectue sous la forme d'un système d'appariement moléculaire, dans lequel le système d'appariement moléculaire contient un composé choisi dans le groupe des acides pentopyranosyl-nucléiques, un acide nucléique avec une ou plusieurs unités acide amino-cyclohexyléthanoïque (CNA), les acides nucléiques peptidiques (PNA) ou un acide nucléique avec une ou plusieurs [2-amino-4-(carboxyméthyl)cyclohexyl]-nucléobases.

2. Système de reconnaissance selon la revendication 1, **caractérisé en ce que** l'unité pentopyranosyle de l'acide pentopyranosyl-nucléique est un ribose, un arabinose, un lyxose ou un xylose.

3. Système de reconnaissance selon la revendication 2, **caractérisé en ce que** l'acide pentopyranosyl-nucléique est un pyranosyl-ARN (p-ARN).

4. Système de reconnaissance selon l'une des revendications 1 à 3, **caractérisé en ce que** l'unité de nucléobase du système d'appariement moléculaire est choisie parmi la purine, la 2,6-diaminopurine, le 6-purinethiol, la pyridine, la pyrimidine, l'adénine, la guanine, l'isoguanine, la 6-thioguanine, la xanthine, l'hypoxanthine, la thymidine, la cytosine, l'isocytosine, l'indole, la tryptamine, la N-phtaloyltryptamine, l'uracile, la caféine, la théobromine, la théophylline, le benzotriazole ou l'acridine.

5. Système de reconnaissance selon une des revendications 1 à 4, **caractérisé en ce que** le pyranosyl-nucléotide est choisi parmi la ribopyranosyladénosine, ribopyranosylguanosine, ribopyranosylthymidine, ribopyranosylcytosine, ribopyranosyltryptamine ou ribopyranosyl-N-phtaloyltryptamine, le ribopyranosyl-uracile ou leurs dérivés 2-amino-4-(carboxyméthyl)ribopyranosyle.

6. Système de reconnaissance selon l'une des revendications 1 à 5, **caractérisé en ce que** la longueur de l'acide pentopyranosyl-nucléique est de 4 à 50 nucléotides.

7. Système de reconnaissance selon l'une des revendications 1 à 6, **caractérisé en ce que** le composant de liaison A est immobilisé sur un support.

8. Système de reconnaissance selon la revendication 7, **caractérisé en ce que** le support est choisi dans le groupe des céramiques, des métaux, des verres, des matières plastiques, des matériaux cristallins ou les couches minces du support des matériaux indiqués, ou des filaments (bio)moléculaires, la cellulose, les protéines de structure.

9. Système de reconnaissance selon la revendication 7 ou 8, **caractérisé en ce que** le composant de liaison A est immobilisé sur un support par une liaison covalente, une liaison quasi-covalente ou une liaison supramoléculaire par association de deux ou plus de deux espèces moléculaires comme des molécules de constitution linéaire choisies dans le groupe des peptides, peptoïdes, protéines, oligo- ou poly-saccharides linéaires, les acides nucléiques et leurs analogues, ou des monomères hétérocycliques, ou des molécules de constitution non linéaire choisies dans le groupe des oligo- ou polysaccharides ramifiés ou des anticorps et de leurs parties fonctionnelles choisies dans le groupe des fragments Fv, des fragments Fv à chaîne simple (scFv) ou des fragments Fab.

10. Système de reconnaissance selon l'une des revendications 7 à 9, **caractérisé en ce que** le composant de liaison A est immobilisé à des positions définies du support, de préférence sous la forme d'une matrice.

11. Système de reconnaissance selon la revendication 10, **caractérisé en ce que** les positions définies du support sont adressées.

12. Système de reconnaissance selon l'une des revendications 7 à 11, **caractérisé en ce que** le composant de liaison A est immobilisé sur une électrode support du support.

13. Système de reconnaissance selon l'une des revendications 1 à 12, **caractérisé en ce que** l'espèce de reconnaissance B est une biomolécule.

14. Système de reconnaissance selon la revendication 13, **caractérisé en ce que** la biomolécule est choisie dans le groupe des peptides, peptoïdes, protéines ou leurs parties fonctionnelles, anticorps ou leurs parties fonctionnelles, fragments Fv, fragments Fv à chaîne simple (scFv) ou fragments Fab, ou constituants cellulaires, lipides, glycoprotéines, constituants de filaments, ou des virus, constituants de virus, capsides, ou viroïdes, ou leurs dérivés, et leurs parties actives, ou bibliothèques de substances telles que des ensembles de composés structurellement différents du groupe des peptides oligomères ou polymères, des peptoïdes, des saccharides et des acides nucléiques.

15. Système de reconnaissance selon l'une des revendications 1 à 14, **caractérisé en ce que** le composant immobilisé A contient différentes positions de liaison pour différentes espèces de reconnaissance B, au moyen duquel différentes espèces de reconnaissance B peuvent se lier au composant de liaison A.

16. Système de reconnaissance selon l'une des revendications 1 à 15, **caractérisé en ce qu'**au moins une autre espèce de reconnaissance B est immobilisée sur le composant de liaison B.

17. Système de reconnaissance selon la revendication 15 ou 16, **caractérisé en ce qu'**il comprend
(a) au moins un composant de liaison A immobilisé avec au moins 2+n positions de liaison différentes pour au moins 2+n espèces de reconnaissance différentes B1, B2 ... Bn et une autre espèce de reconnaissance B(n+3) différente des espèces de reconnaissance B1, B2... Bn, qui est immobilisée sur le composant de liaison A immobilisé, et
(b) au moins (n+3) espèces de reconnaissance B1, B2 ... B(n+3) différentes,
dans lequel n représente un nombre entier de 0 à 20, de préférence de 0 à 10, en particulier de 0 à 5, surtout 0 ou 1.

18. Système de reconnaissance selon la revendication 17, **caractérisé en ce que** les espèces de reconnaissance B1, B2 ... Bn proviennent d'une bibliothèque de substances.

19. Système de reconnaissance selon la revendication 17 ou 18, **caractérisé en ce que** la structure de l'espèce de reconnaissance B(n+3) est connue.

20. Système de reconnaissance selon l'une des revendications 15 à 19, **caractérisé en ce que** les différentes espèces de reconnaissance B reconnaissent le même substrat S.

21. Système de reconnaissance selon l'une des revendications 1 à 20, **caractérisé en ce que** le substrat est choisi dans le groupe des médicaments et des substances phytosanitaires, les métabolites, les substances de messagers physiologiques, les dérivés de structures directrices, les substances qui sont produites ou produites en plus grande quantité dans le corps humain ou animal dans le cas de modifications pathologiques, ou les analogues d'états de transition, ou les peptides, peptoïdes, protéines comme des récepteurs ou des parties fonctionnelles, des anticorps ou des parties fonctionnelles de ceux-ci, des fragments Fv, des fragments Fv à chaîne simple (scFv) ou des fragments Fab, ou des constituants cellulaires, des lipides, des glycoprotéines, des constituants de filaments, ou des virus, constituants de virus, capsides, ou viroïdes, ou leurs dérivés, ou des monomères hétérocycliques, ou des molécules de constitution non linéaire dans le groupe des oligo- ou polysaccharides ramifiés ou des bibliothèques de substances telles que des ensembles de composés structurellement différents, des peptides oligomères ou polymères, des peptoïdes, des saccharides, des acides nucléiques, des esters, acétals ou des monomères hétérocycliques, des lipides, stéroïdes ou des structures cibles pour des produits pharmaceutiques, des récepteurs de médicaments, des canaux ioniques dépendants du potentiel, des transporteurs, des enzymes ou des unités de biosynthèse de micro-organismes.

22. Système de reconnaissance selon l'une des revendications 1 à 21, **caractérisé en ce qu'**il représente un immunoessai.

23. Procédé d'identification d'un substrat S dans un échantillon à l'aide du système de reconnaissance selon l'une des revendications 1 à 22, **caractérisé en ce que**
(a) on met en contact avec l'échantillon une espèce de reconnaissance B qui reconnaît le substrat,
(b) on met en contact en même temps ou successivement avec une espèce de reconnaissance B immobilisé, et
(c) on détecte la formation d'un complexe du composant de liaison immobilisé A, de l'espèce de reconnaissance B et du substrat S.

24. Procédé selon la revendication 23, **caractérisé en ce que** la formation du complexe est régulée par des paramètres physiques comme la température, les sels, les solvants, des procédés électrophorétiques.

25. Procédé selon la revendication 23 ou 24, **caractérisé en ce que** le complexe est détecté par un marquage radioactif ou de fluorescence, un marquage enzymatique, un marquage redox, un marquage par centrifugation de l'espèce de reconnaissance B, ou via le complexe lui-même, par des procédés d'électrode, des procédés chimiques, des procédés redox dans l'environnement ou à l'électrode ou via une grandeur de mesure physique, une mesure d'impédance ou une mesure en courant continu.

26. Procédé selon l'une des revendications 23 à 25, **caractérisé en ce qu'**on isole dans une autre étape le complexe de l'espèce de reconnaissance B et du substrat S.

27. Procédé selon la revendication 26, **caractérisé en ce qu'**on isole le complexe de l'espèce de reconnaissance B et du substrat S après congélation de l'équilibre de liaison ou de la réticulation covalente de l'espèce de reconnaissance B et du substrat S.

28. Utilisation du système de reconnaissance selon l'une des revendications 1 à 22 pour trouver un substrat S, pour le diagnostic, pour la préparation d'un catalyseur et/ou pour la préparation d'un composant électronique, pour trouver ou pour optimiser et/ou pour préparer un médicament ou une substance phytosanitaire.
